(19) Europäisches Patentamt
European Patent Office
Office européen des brevets

(11) Veröffentlichungsnummer: **0 041 655**
**B1**

(12) # EUROPÄISCHE PATENTSCHRIFT

(45) Veröffentlichungstag der Patentschrift: **25.07.84**

(51) Int. Cl.³: **C 07 D501/20**, C 07 D501/02// C07D417/12

(21) Anmeldenummer: **81104087.2**

(22) Anmeldetag: **27.05.81**

(54) **2-(2-Aminothiazol-4-yl)-N-(imidazolidin und perhydro-1,3-diazin-2-on-l-yl)-2-iminoacetylcephalosporinen der syn-Form und Verfahren zu deren Herstellung.**

(30) Priorität: **06.06.80 DE 3021373**

(43) Veröffentlichungstag der Anmeldung: **16.12.81 Patentblatt 81/50**

(45) Bekanntmachung des Hinweises auf die Patenterteilung: **25.07.84 Patentblatt 84/30**

(84) Benannte Vertragsstaaten: **BE CH DE FR GB IT LI**

(56) Entgegenhaltungen: **EP-A- 0 004 956**

(73) Patentinhaber: **BAYER AG**
**Konzernverwaltung RP Patentabteilung**
**D-5090 Leverkusen 1 Bayerwerk (DE)**

(72) Erfinder: **König, Hans-Bodo, Dr.**
**Herbertskaternberg 10**
**D-5600 Wuppertal 1 (DE)**
Erfinder: **Metzger, Karl Georg, Dr.**
**Pahlkestrasse 15**
**D-5600 Wuppertal 1 (DE)**

**EP 0 041 655 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

In der DE-OS 2 818 263 ist neben weiteren die Verbindung der Formel (I)

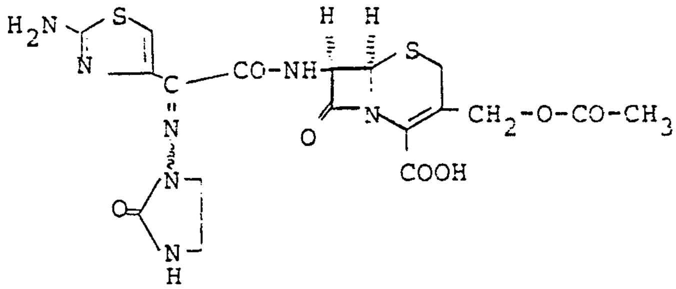

(I)

beschrieben.

Sie kann sowohl in der syn-Form (der Imidazolidinonring ist zum Cephemgerüst hin gerichtet) als auch in der anti-Form existieren.

Nach dem in der DE-OS 2 818 263 beschriebenen Verfahren zur Herstellung dieser Verbindungen wird aber stets die zur Umsetzung mit der 7-Aminocephalosporansäure verwendete Säure, soweit sie eine Aminogruppe besitzt, zuvor an der Aminogruppe mit einer Schutzgruppe versehen, z. B.

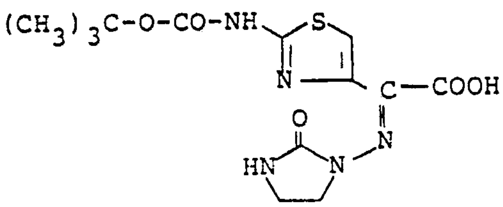

(II)

Nach der Kupplung dieser Säure mit der 7-Aminocephalosporansäure wird dann mittels Trifluoressigsäure gleichzeitig die Schutzgruppe abgespalten und, unter der Einwirkung dieser starken Säure, die anti-Form teilweise in die syn-Form umgewandelt. Unter der Einwirkung der Trifluoressigsäure stellt sich zwischen der syn- und der anti-Form ein Gleichgewicht ein, in dem beide Formen etwa zu gleichen Teilen enthalten sind. Daß es sich um ein Gleichgewicht handelt, erkennt man daran, daß auch die reine anti-Form in Trifluoressigsäure in wenigen Augenblicken das gleiche Gemisch ergibt. Außerdem fallen die Cephalosporine gemäß DE-OS 28 18 263 stets in amorpher Form an.

Es wurde nun überraschenderweise gefunden, daß man reine und kristalline syn-Cephalosporine der allgemeinen Formel (III)

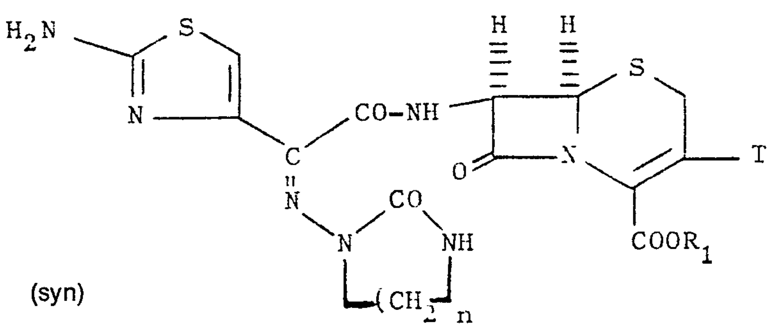

(syn)

(III)

gerade in Gegenwart einer starken Säure z. B. Salzsäure und in Abwesenheit von Basen direkt herstellen kann.

Die reine syn-Form hat sich als wesentlich wirksamer herausgestellt, als die anti-Form.

Die Erfindung betrifft daher Cephalosporine in reiner, kristalliner Form der allgemeinen Formel (III), in der

n 2 oder 3

$R^1$ Natrium oder, wenn ein anderer Molekülteil eine positive Ladung enthält, eine negative Ladung darstellt und

T $-CH_2-O-CO-CH_3$, $-CH_2-O-CO-NH_2$ oder

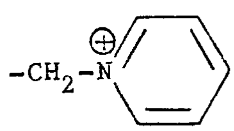

bedeuten. Die Erfindung betrifft weiter ein Verfahren zur Herstellung solcher Cephalosporine, das dadurch gekennzeichnet ist, daß man ein entsprechendes 7-Aminocephalosporansäure-Derivat mit einem Säurehalogenid der Formel (IV)

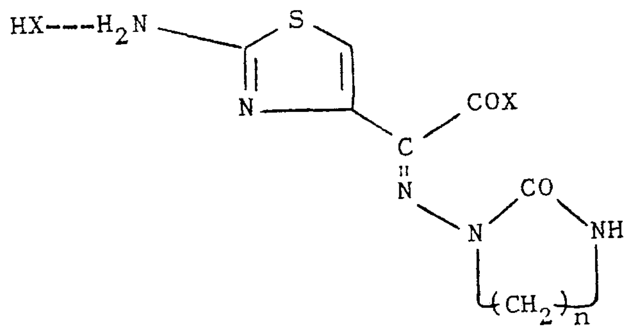

(IV)

in der

n die oben angegebene Bedeutung hat und

X Chlor darstellt

umsetzt.

Die Umsetzung wird vorzugsweise in einem inerten Lösungsmittel, z. B. Tetrahydrofuran (THF) durchgeführt. Dabei wird im allgemeinen bei einer Temperatur von − 80 bis + 25 °C und vorzugsweise von − 50 bis 0 °C gearbeitet.

Vorzugsweise werden die Cephalosporansäurederivate vor der Umsetzung mit den Säurechloriden der Formel (IV) an der Aminogruppe und der Carboxylgruppe silyliert, z. B. werden Trimethylsilyl-, Triethylsilyl oder andere Silylreste eingeführt.

Die Reaktion wird ferner in Abwesenheit von Basen durchgeführt. Dies ist besonders deshalb erforderlich, weil die Säurechloride (IV) basenempfindlich sind. Unter diesen Reaktionsbedingungen aber muß es als besonders überraschend angesehen werden, daß die gewünschten Verbindungen in der reinen syn-Form erhalten werden, da, wie oben beschrieben, unter dem Einfluß starker Säure, wie der Trifluoressigsäure aus der anti-Verbindung ein Gleichgewichtsgemisch aus syn- und anti-Verbindung erhalten wird.

Die als Ausgangsprodukt eingesetzten syn-Säurechloride (IV) sind neu und stellen einen weiteren Gegenstand der vorliegenden Erfindung dar. Diese Säurechloride können auf folgende Weise hergestellt werden:

Durch Umsetzung der Aminothiazolylglyoxylsäure

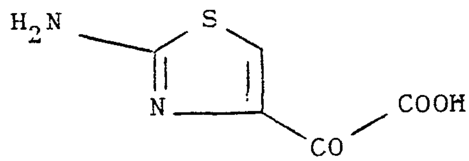

mit der Hydrazinverbindung

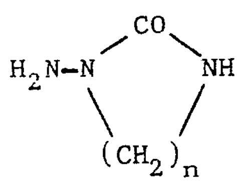

worin n die oben angegebene Bedeutung hat, erhält man überraschenderweise die syn-Säuren der Formel (V)

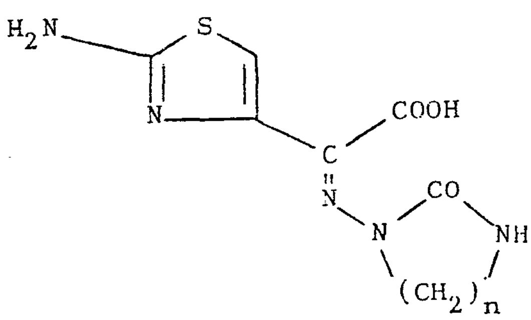

(V)

worin n die oben angegebene Bedeutung hat.

Daß hierbei die syn-Form gebildet wird, ist deshalb überraschend, weil bei der Umsetzung einer entsprechenden Aminothiazolylglyoxylsäure, die lediglich an der Aminogruppe eine Schutzgruppe (BOC-Rest) trägt, mit einer entsprechenden Hydrazinverbindung die anti-Form

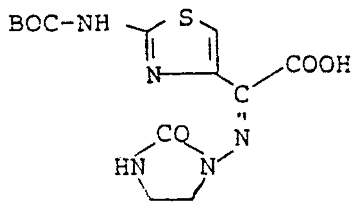

(anti)

erhalten wird.

BOC = $(CH_3)_3C—O—CO—$

Syn- und anti-Form kann man anhand des NMR-Spektrums unterscheiden. Beispielsweise die $—CH_2—CH_2$-Gruppe im Imidazolidinonteil gibt in der syn-Form zwei Multipletts, während die anti-Form ein Pseudosingulett zeigt.

Die syn-Säure der Formel (V) mit n = 2 läßt sich nur unter bestimmten Bedingungen mit Thionylchlorid in das syn-Säurechlorid-hydrochlorid der Formel (IV) überführen. Zu diesen Bedingungen gehört, daß man das in der Säure der Formel (V) enthaltene Kristallwasser nicht entfernt und zum Lösungsmittel noch soviel Wasser vor der Zugabe des Thionylchlorids hinzufügt, daß im Reaktionsgemisch etwa 2-4 Moläquivalente Wasser, bezogen auf die Syn-Säure (V) enthalten sind. Die syn-Säure der Formel (V) mit n = 3 läßt sich mit Thionylchlorid getrocknet (0,4 Moläquivalente $H_2O$) oder ungetrocknet (1,7 Moläquivalente $H_2O$) in das syn-Säure-chlorid-hydrochlorid der Formel (IV) überführen.

Als Lösungsmittel wird Acetonitril oder ein niederer Nitroaliphat, z. B. Nitromethan verwendet. Das Thionylchlorid muß bei dieser Umsetzung in großem Überschuß eingesetzt werden. Das Molverhältnis von Säure (V) zu Thionylchlorid beträgt mindestens 1 : 3, vorzugsweise 1 : 12. Man kann auch Thionylchlorid selbst als Verdünnungsmittel verwenden, erhält dann aber das Säurechlorid-hydrochlorid nicht in derselben Reinheit.

Die Chlorierung der Säuren (V) erfolgt im allgemeinen bei einer Temperatur von 0 bis 30 °C, vorzugsweise bei 15 bis 25 °C.

2-Aminothiazol-Reste können auch als 2-Imino-4-thiazolin-Reste bezeichnet oder geschrieben werden.

Figur 1 zeigt das IR-Spektrum der Verbindung gemäß Beispiel 3b in Nujol.

Figur 2 zeigt das IR-Spektrum der Verbindung gemäß Beispiel 4 in Nujol.

## Beispiel 1

2-(2-Imino-4-thiazolin-4-yl)-N-(imidazolidin-2-on-1-yl)-2-iminoessigsäure (syn-Form)

Zur Lösung von 7,6 g 1-Amino-2-oxoimidazolidin in 90 ml Wasser gibt man 10 g 2-(2-Imino-4-thiazolin-4-yl)-glyoxylsäure und rührt bei 20 °C über Nacht. Das auskristallisierte Produkt wird abgesaugt, mit Wasser gewaschen und im Exsiccator getrocknet.

Ausbeute : 7,2 g, Fp. : > 240 °C

$C_8H_9N_5O_3SxH_2O$

ber. : C 35,2 H 4,0 N 25,6 S 11,7

gef. : C 35,2 H 4,1 N 25,3 S 11,5

NMR (100 MHz ; δ in $d_6$-DMSO/$CD_3OD$) 6,88 (s ; Thiazolin-5-H) ; 3,9-3,7 (m ; $N—CH_2—CH_2—N$), 3,5-3,27 (m ; $N—CH_2—CH_2—N$).

## Beispiel 2a

2-(2-Imino-4-thiazolin-4-yl)-N-(imidazolidin-2-on-1-yl)-2-iminoessigsäurechlorid-hydrochlorid

4,65 g 2-(2-Imino-4-thiazolin-4-yl)-N-(imidazolidin-2-on-1-yl)-2-iminoessigsäure syn-Form bezüglich Carboxylgruppe (Korngröße zu 98 % < 5 μ ; die Substanz enthielt 1,8 Moläquivalente Wasser) werden in 96 ml Acetonitril (Wassergehalt 0,30 %) suspendiert und unter trockenem $N_2$ 16 ml Thionylchlorid bei 20 °C zugegeben. Man rührt 1,5 Stunden bei 20 °C, saugt dann den ausgefallenen kristallinen gelben Niederschlag unter Feuchtigkeitsausschluß ab und wäscht zweimal mit absolutem Acetonitril. Nach Trocknen im Exsiccator betrug die Ausbeute : 5,0 g (ca. 100 % d. Th.)

IR-Spektrum (Nujol) (Carbonylbereich) : 1 810 (CO—Cl), 1 660-1 685, 1 625 (Fingerprintgebiet) : 975, 890, 855, 800, 775 und 725 $cm^{-1}$.

Die Substanz ist sehr feuchtigkeitsempfindlich. Bei absolutem Ausschluß von Feuchtigkeit und bei einer Temperatur von 0 °C war sie auch rach mehreren Wochen unverändert.

## Beispiel 2b

Die Mischung von 5 g der in Beispiel 2a verwendeten Säure, 100 ml Nitromethan (Wassergehalt 0,17 %) und 50 ml Nitromethan (Wassergehalt 0,02 %) sowie 5,7 ml Thionylchlorid wurde unter Feuchtigkeitsausschluß 24 Stunden bei 20 °C gerührt. Der ausgefallene Niederschlag wurde dann abgesaugt, mit abs. Nitromethan gewaschen und im Exsiccator getrocknet.

Ausbeute : 5,3 g

IR-Spektrum : identisch mit dem von Beispiel 2a.

## Beispiel 2c

Verwendet man jeweils 0,5 g der in Beispiel 2a eingesetzten Säure, als Lösungsmittel 1-Nitropropan (15 ml) und 1,72 ml Thionylchlorid, so erhält man, nach Versuchsausführung, wie in Beispiel 2b, das Säurechlorid-hydrochlorid von Beispiel 2a in nahezu quantitativer Ausbeute, wenn der Wassergehalt des 1-Nitropropans 0,19 % beträgt. Enthält das verwendete 1-Nitropropan nur 0,04 % Wasser, so entsteht praktisch kein Säurechlorid (laut IR-Spektrum).

## Beispiel 2d

Verwendet man jeweils 0,5 g der in Beispiel 2a eingesetzten Säure, als Lösungsmittel 15 ml Nitromethan und 0,6 ml Thionylchlorid, so erhält man nach 24-stündigem Rühren bei 20 °C das Säurechlorid-hydrochlorid von Beispiel 2a dann in sehr guter Ausbeute, wenn der Wassergehalt des Nitromethans 0,17 % beträgt. Bei einem Wassergehalt des Lösungsmittels von 0,02 % tritt im IR-Spektrum des Reaktionsproduktes keine Bande bei 1 810 $cm^{-1}$ auf, d. h., es wurde praktisch kein Säurechlorid gebildet.

## Beispiel 2e

Rührt man die Mischung von 10 g der in Beispiel 2a eingesetzten Säure, 300 ml 2-Nitropropan (Wassergehalt : 0,19 %) und 34,4 ml Thionylchlorid 24 Stunden bei 20 °C, so erhält man das Säurechlorid-hydrochlorid von Beispiel 2a als Niederschlag und in nahezu quantitativer Ausbeute.

Erniedrigt man jedoch bei diesem Versuch lediglich die Menge des 2-Nitropropans auf 150 ml (bei gleichem Wassergehalt von 0,19 %), so zeigt das ausgefallene Reaktionsprodukt im IR-Spektrum keine Säurechloridbande bei 1 810 $cm^{-1}$.

## Beispiel 3a

7-[(2-Aminothiazol-4-yl)-N-(imidazolidin-2-on-1-yl)-2-iminoacetamido]-3-acetoxymethyl-3-cephem-4-carbonsaures-Natrium (syn-Form) (amorph)

Die Mischung von 16,1 g 7-Aminocephalosporansäure (94 %ig) und 110 ml trockenem Tetrahydrofuran wird unter Rühren bei 20 °C mit 32 ml Bis-trimethylsilylacetamid versetzt. Sie geht unter geringer Temperaturerhöhung in etwa 2 Minuten in Lösung. Diese Lösung wird auf − 50 °C gekühlt und unter $N_2$ 20,2 g des gemäß Beispiel 2a hergestellten Säurechlorid-hydrochlorids zugegeben. Man rührt noch 10 Minuten im Kältebad, wobei die Temperatur auf − 65 °C sinkt. Dann wird das Kältebad entfernt. Nach etwa 20 Minuten ist die Temperatur auf 0°-5 °C gestiegen und alles in Lösung gegangen. Nun gießt man die Reaktionslösung unter Rühren in 3 Liter Ether und rührt in einem offenen Gefäß etwa 30 Minuten. Der dann ausgefallene Niederschlag besteht aus dem amorphen Hydrochlorid des in der Überschrift genannten Cephalosporins (syn-Form). Der Niederschlag wird abgesaugt, wird mit Ether gewaschen und anschließend in 20 Minuten portionsweise in 1,2 Liter Wasser von 0°-5° unter Rühren eingetragen. Durch gleichzeitiges Zugeben von festem $NaHCO_3$ oder Natronlauge wird der pH der Mischung auf etwa 3 gehalten. Anschließend wird der pH auf 3,2 eingestellt, 15-20 Minuten noch gerührt und dann ein etwas geliger Niederschlag, der im wesentlichen Verunreinigungen enthält, abgesaugt. Dabei wird das Filtrat durch entsprechende Zugabe von 2n Natronlauge bei pH 5 gehalten und gekühlt. Der gelige Niederschlag wird gründlich mit Eiswasser (200 ml) gewaschen. Die vereinigten wäßrigen Filtrate werden auf pH 6,5 eingestellt und gefriergetrocknet. Man erhält so das amorphe Natriumsalz des in der Überschrift genannten Cephalosporins (syn-Form).

Die Ausbeute schwankt bei mehreren Ansätzen zwischen 24,2-32,7 g.

Der Gehalt an syn-Form beträgt nach HPLC etwa 75 %. Anti-Form ist nur zu $\leq$ 1 % vorhanden. Das Rohprodukt enthält NaCl (ca. 5 %), Acetamid (etwa 1 Moläquivalent) enthält NaCl (ca. 5 %), Acetamid (etwa 1 Moläquivalent) und Wasser (etwa 1-2 Moläquivalente).

NMR (100 MHz ; δ in $CD_3OD$-$d_6DMSO$) 6,78 (s ; Thiazolin 5-H) ; 5,75 (α ; β-Lactam ; I = 4,7) ; 5,1 (α ; β-Lactam ; I = 4,7).

Beispiel 3b

7-[(2-Aminothiazol-4-yl)-N-(imidazolidin-2-on-1-yl)-2-iminoacetamido]-3-acetoxymethyl-3-cephem-4-carbonsaures-Natrium.
(syn-Form) (kristallin) (Ethanolat) (in Wasser leichtlösliche Form).

62,6 g eines gemäß Beispiel 3a hergestellten, amorphen syn-Cephalosporin-Natriumsalzes werden in 250 ml Wasser bei 20 °C gelöst. Zu dieser Lösung gibt man zunächst unter Umschwenken 600 ml Ethanol und, nachdem durch Anreiben die Kristallisation in gang gesetzt wurde, weitere 400 ml Ethanol. Man läßt die Suspension 5 Minuten bei 20 °C stehen, saugt ab und wäscht mit wäßrigem Ethanol (W./A = 1/4) nach. Ausbeute : 40,4 g.

Die Substanz ist kristallin und enthält nach Analyse und NMR-Spektrum etwa 1 Mol Kristall-Wasser und etwa 1 Mol Kristall-Ethanol

NMR ($^1$H-360 MHz ; in $D_2O$ ; δ in ppm ; I in Hz)

3,210 und ~ 3,5 (—A—$CH_2$— ; $I_{AB}$ = (−) 18,0)
5,040 (β-Lactam ; I = 4,7)
5,710 (β-Lactam ; I = 4,7)
4,727 und 4,560 (—$CH_2$—O—CO— ; $I_{AB}$ = (−) 12,6)
1,910 (—O—CO—$CH_3$)
6,810 (Thiazol)
3,737 und 3,393 (—N—$CH_2$—$CH_2$—N—)
3,460 ($CH_2$EtOH)
0,982 ($CH_3$EtOH)

NMR ($^{13}$C-25,2 MHz ; δ in ppm, rel. z. TMS = 0 ; in $D_2O$)

| C-Atome | (ppm) |
|---|---|
| 2 | 26,608 |
| 3 | 117,126 |
| 4 | 132,303 |
| 6 | 58,244 |
| 7 | 59,622 |
| 8 | 167,582 |
| 10 | 170,820 |
| $CH_2$ an $C_3$ | 64,974 |
| CO von OAc | 174,564 |
| $CH_3$ von OAc | 21,181 |
| $CH_2$ d zur N(N) | 46,635 |
| $CH_2$ d zur NH | 37,867 |
| CO (Harnstoff) | 161,631 |
| $CH_2$ von EtOH | 57,890 |
| $CH_3$ von EtOH | 17,784 |

d-Werte und die dazugehörigen Intensitäten einer Röntgenbeugungsanalyse nach Debye-Scherrer (Nachweis der Kristallinität) :

(Siehe Tabelle, Seite 7 f.)

| | | | | |
|---|---|---|---|---|
| | 4.04 | 3.97 | 15.13 | 17.48 |
| 590/80 | 100 | 100 | 50 | 2 |

| λ = 1.5418 Å | | dÅ | I | hkl | dÅ | I | hkl | dÅ | I |
|---|---|---|---|---|---|---|---|---|---|
| Filter: Ni | | 17.48 | 2 | | 3.81 | 2 | | 2.107 | 2 |
| | | 15.13 | 50 | | 3.66 | 25 | B | (2.053 | 5 |
| | | 13.29 | 1 | | 3.53 | 10 | | (2.006 | 5 |
| Intensität: | | 9.57 | 1 | | 3.42 | 5 | | | |
| geschätzt | | 8.68 | 1 | | 3.24 | 15 | | | |
| | | 8.01 | 25 | | 3.08 | 10 | | | |
| | | 7.63 | 10 | B | (2.947 | 15 | | | |
| B = Band | | 6.87 | 5 | | (2.900 | 20 | | | |
| | | 6.12 | 25 | | 2.787 | 3 | | | |
| | | 5.85 | 10 | | 2.666 | 15 | | | |
| | | 5.54 | 5 | | 2.559 | 10 | | | |
| | | 4.74 | 30 | | 2.473 | 8 | | | |
| | | 4.51 | 15 | | 2.353 | 1 | | | |
| | B | (4.04 | 100 | | 2.269 | 20 | | | |
| | | (3.97 | 100 | | 2.180 | 5 | | | |

Beispiel 3c

7-[(2-Aminothiazol-4-yl)-N-(imidazolidin-2-on-1-yl)-2-iminoacetamido]-3-acetoxymethyl-3-cephem-4-carbonsaures-Natrium (syn-Form) (kristallin).

67,3 g 7-Aminocephalosporansäure (94 %ig), 450 ml trockenes Tetrahydrofuran und 134 ml Bis-trimethylsilylacetamid werden in dieser Reihenfolge unter trockenem $N_2$ unter Rühren zusammengegeben. Nachdem eine klare Lösung (in wenigen Minuten) entstanden ist, wird auf − 50 °C abgekühlt und 84,5 g des gemäß Beispiel 2a hergestellten Säurechlorid-hydrochlorids unter Rühren zugegeben. Man rührt dann noch etwa 10 Minuten bei der gleichen Temperatur, entfernt dann das Kältebad und läßt die Temperatur von alleine auf etwa 0° ansteigen. Wenn dann alles in Lösung gegangen ist, wird in die Mischung von 4 000 ml Tetrahydrofuran und 3 ml Wasser unter Rühren eingegossen und der sich ausscheidende Niederschlag abgesaugt. Dieser Niederschlag wird nutschenfeucht in 2 000 *) ml Eiswasser unter Rühren und weiterer Kühlung mit Eis eingetragen und dabei der pH mittels 2n Natronlauge bei etwa 5 bis 5,5 gehalten und zum Schluß auf 6,5 eingestellt. Die erhaltene Lösung wird dann aus einem Bad von etwa 30-35 °C auf ein Volumen von 800 bis 1 000 ml mittels eines Rotationsverdampfers im Vakuum eingeengt und dann unter Rühren nach und nach mit etwa 3 000 ml Ethanol versetzt. Dabei wird zwischendurch mit kristallinem Material angeimpft. Anschließend rührt man die erhaltene Suspension noch etwa 30 Minuten, saugt ab, wäscht mit Ethanol/Wasser (4/1) und Ethanol und trocknet im Vakuum.

Ausbeute : 109,4 g (74 % d. Th. bez. a. $C_{18}H_{18}N_7NaO_7S_2xH_2OxC_2H_5OH$) (syn-Form ; kristallin)

Das IR- und NMR-Spektrum ist identisch mit der im Beispiel 3b beschriebenen Substanz. Durch Trocknen, beispielsweise im Umlufttrockenschrank bei 40°-70 °C, kann man den im Kristall erhaltenen Ethylalkohol entfernen. Die Substanz behält daher den kristallinen Zustand und die syn-Form. Das IR-Spektrum der festen Substanz ändert sich geringfügig.

Beispiel 3d

7-[(2-Aminothiazol-4-yl)-N-(imidazolidin-2-on-1-yl)-2-iminoacetamido]-3-acetoxymethyl-3-cephem-4-carbonsaures-Natrium (syn-Form) (kristallin).

Die Mischung von 6 g (0,022 Mole) 7-Aminocephalosporansäure, 60 ml Acetonitril (ü. Mol.-sieb getr.) und 5,4 ml (0,026 Mole) Hexamethyldisilazan wird unter Rühren in ein auf 100 °C vorgeheiztes Ölbad getaucht und dabei ein schwacher $N_2$-Strom über die bald unter Rückfluß kochende Suspension geleitet. Nach etwa 15 Min. hat sich eine klare Lösung gebildet und nach insgesamt etwa 1 Std. ist im Dampfraum über dem Reaktionsgemisch mit Indikatorpapier kein Ammoniak mehr nachweisbar. Nun wird auf 30 °C abgekühlt und unter Rühren mittels einer Ölpumpe unter Vorschaltung einer Kühlfalle (fl. $N_2$) die

*) Als Alternative kann man auch in weniger Wasser (etwa 1 000 ml) lösen und anschließend etwa 3 000 ml Ethanol zugeben. Auch dann erhält man das Cephalosporin in entsprechender Reinheit und Ausbeute.

Reaktionslösung bis auf ein Volumen von etwa 30 ml eingeengt, wobei der Reaktionskolben zur Ergänzung des Wärmeverlustes in ein Wasserbad von Raumtemperatur getaucht wird. Die verbleibende Reaktionslösung wird dann in Eis/Wasser auf 5 °C abgekühlt. 5,88 g (0,019 Mole) des Säurechloridhydrochlorids :

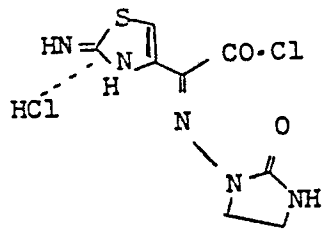

werden dann auf einmal zugegeben. Die Innentemperatur steigt dabei auf 26 °C an. Wenn die Innentemperatur wieder auf 20 °C gesunken ist, wird das Eisbad entfernt und noch 3 Std. und 40 Min. bei Raumtemperatur weitergerührt. Dann wird das Reaktionsgemisch in 600 ml Ether (Ferak, $H_2O < 0,1$ %), der in einem offenen Gefäß gerührt wird, eingegossen, 30 Min. nachgerührt, der gebildete Niederschlag durch Glasfritte abgesaugt, einmal mit Ether auf der Nutsche gewaschen, abgepreßt und von der Nutsche in 60 ml Eiswasser eingetragen, wobei man gleichzeitig durch entsprechendes Zugeben von 2 n Natronlauge den pH zwischen 5 und 7 hält. Dann rührt man noch 10 Min. bei pH 7,5 nach und zerteilt dabei die Klumpen des Filterkuchens. Man filtriert eine kleine Menge Ungelöstes (Verunreinigung) ab, stellt das klare Filtrat (falls nötig) nochmals auf pH 7,5 ein (Volumen jetzt ca. 120 ml) und gibt dann 350 ml EtOH zu, reibt an, gibt weitere 150 ml EtOH zu, rührt bei Raumtemperatur ca. 30 Min. und saugt dann das kristalline Natriumsalz ab. Man wäscht auf der Nutsche 2 × mit 20 ml $EtOH/H_2O = 5/1$ und dann 1 × mit 20 ml EtOH.

Ausbeute (nach Trocknen i. Exsicc. ü. $P_2O_5$) = 4,7 g (45 % d. Th.) (kristallin).

Reinigung des kristallinen Rohproduktes

4,5 g des Rohproduktes werden in 45 ml Wasser bei Raumtemperatur unter Rühren gelöst (geht rasch) und dann zur klaren Lösung sofort 120 ml EtOH gegeben. Dabei scheiden sich wenige braune Flocken aus. Diese wurden durch Filtration entfernt. Man wäscht mit etwa EtOH nach. In das Filtrat gibt man dann unter Rühren weitere 280 ml EtOH, reibt an und rührt bei Raumtemperatur noch 40 Min. Dann saugt man ab, wäscht 2 × mit 10 ml $EtOH/H_2O = 5/1$ und 1 × mit EtOH.

Ausbeute (Exsicc., $P_2O_5$) : 3,2 g (kristallin) (entspr. 32 % d. Th. Gesamtausb.).

NMR (250 MHz ; δ in $CD_3OD/D_2O$) 6,77 (s, Thiazolin-5-H) ; 5,77 (d ; 1H ; β-Lactam) ; 5,07 (d ; 1H ; β-Lactam) ; 3,85-3,22 (m ; 6H ; C-2 ; Imidazolidinon).

Beispiel 4

7-[(2-Aminothiazol-4-yl)-N-(imidazolidin-2-on-1-yl)-2-iminoacetamido]-3-acetoxymethyl-3-cephem-4-carbonsaures-Natrium (syn-Form) (Kristallin) (in Wasser schwerlösliche Form).

20 g des unter Beispiel 3b beschriebenen in Wasser leichtlöslichen, kristallinen und ethanolhaltigen Natriumsalzes werden unter Rühren in 100 ml Wasser von 20 °C gelöst und diese Lösung nach Animpfen mit der in Wasser schwerlöslichen Form 30 Minuten weiter gerührt. Dann hat sich die in Wasser schwerlösliche, kristalline Form ausgeschieden. Der Niederschlag wird abgesaugt und dem Exsiccator über $P_4O_{10}$ getrocknet. Die Substanz enthält Ethanol, aber etwa 2 Moläquivalente Wasser.

Ausbeute : 11,65 g.

NMR (250 MHz ; δ in $d_6$-DMSO) 9,82 (d, J 7,5 Hz, C—CO—NH) ; 7,5 (s N—CO—NH) ; 7,5 (d, $H_2N$-Thiazol) ; 6,73 (s, Thiazol-5—H) ; (dd, J 5 Hz u. 7,5 Hz, $C_7$—H) ; 5,03 (d, 5 Hz, $C_6$—H) ; 5,01 u. 4,79 (AB, J 12,5 Hz, $CH_2$—O—CO) ; 3,9-3,2 (m, S—$CH_2$, N—$CH_2$—$CH_2$—N, $H_2O$) ; 2,02 (s, —CO—$CH_3$).

d-Werte und die dazugehörigen Intensitäten einer Röntgenbeugungsanalyse nach Debye-Scherrer (Nachweis der Kristallinität) :

(Siehe Tabelle, Seite 9 f.)

| | 9.61 | 4.35 | 3.70 | 16.99 |
|---|---|---|---|---|
| 598/80 | 100 | 70 | 70 | 5 |

| | dÅ | I | hkl | dÅ | I | hkl | dÅ | I |
|---|---|---|---|---|---|---|---|---|
| λ = 1.5418 Å | 16.99 | 5 | | 3.52 | 25 | | 2.179 | 1 |
| | 9.61 | 100 | | 3.41 | 15 | | 2.150 | 15 |
| Filter: Ni | 8.53 | 10 | | 3.27 | 20 | | | |
| | 7.44 | 20 | | 3.11 | 15 | | | |
| | 6.86 | 3 | | 2.956 | 15 | | | |
| Intensität: geschätzt | 6.42 | 15 | | 2.864 | 15 | | | |
| | B (5.64 | 20 | | 2.814 | 3 | | | |
| B = Band | (5.51 | 15 | | 2.744 | 10 | | | |
| | 5.04 | 25 | | B (2.700 | 8 | | | |
| | 4.75 | 40 | | (2.648 | 8 | | | |
| | 4.52 | 10 | | 2.549 | 5 | | | |
| | 4.35 | 70 | | 2.467 | 20 | | | |
| | 4.00 | 60 | | 2.381 | 10 | | | |
| | 3.90 | 30 | | 2.312 | 8 | | | |
| | 3.70 | 70 | | 2.259 | 8 | | | |

## Beispiel 5

7-[(2-Aminothiazol-4-yl)-N-(imidazolidin-2-on-1-yl)-2-iminoacetamido]-3-aminocarbonyloxymethyl-3-cephem-4-carbonsaures-Natrium (syn-Form) (kristallin).

Die Mischung von 1,68 g 7-Amino-3-aminocarbonyloxymethyl-3-cephem-4-carbonsaures-Natrium, 20 ml Tetrahydrofuran und 0,42 ml Trifluoressigsäure wird 20 Min. bei 20 °C gerührt. Dann werden 20 ml Acetonitril und danach 6,2 ml Bis-trimethylsilylacetamid zugegeben und weitere 50 Min. gerührt. Die Mischung wird anschließend auf − 50 °C abgekühlt, 1,77 g des Säurechlorid-hydrochlorids vom beispiel 2a zugegeben und dann 10 Min. bei − 50 °C gerührt. Nach Entfernen des Kältebades steigt die Temperatur in 30 Min. bis auf + 10 °C. Die Reaktionsmischung wird dann in 200 ml Ether gegossen und 10 Min. gerührt. Der ausgefallene Niederschlag wird dann abgesaugt, mit Ether gewaschen und in 20 ml Eiswasser unter Zusatz von 1 n Natronlauge bei pH 6,5 gelöst. Mab filtriert geringe Mengen verunreinigungen ab und versetzt das Filtrat (40 ml) nach und nach mit insgesamt 200 ml EtOH. Dabei fällt das natriumsalz als kristalliner Niederschlag aus.

Ausbeute : 1,8 g.

NMR (250 MHz ; δ ; in $CD_3OD/D_2O$) : 6,85 (s ; 1H ; Thiazolin-5) ; 5,8 (d ; 1H ; β-Lactam ; J = 5 Hz) ; 5,1 (d ; 1H ; β-Lactam ; J = 5 Hz) ; 4,82-4,59 (AB ; $CH_2OCONH_2$ ; unter Austauschbaren) ; 3,86 (t ; 2H ; $=N-N-CH_2$ ; J ≅ 8 Hz) ; 3,59-3,24 (m ; 4H ; $CH_2-NH$ u. C-2).

Das 7-Amino-3-aminocarbonyloxymethyl-3-cephem-4-carbonsäure-Natrium wurde aus 7-Phenylacetylamino-3-aminocarbonyloxymethyl-3-cephem-4-carbonsaurem-Natrium durch enzymatische Abspaltung des Phenylacetylrestes hergestellt.

## Beispiel 6

7-[(2-Aminothiazol-4-yl)-N-(perhydro-1,3-diazin-2-on-1-yl)-2-iminoacetamido]-3-pyridiniomethyl-3-cephem-4-carbonat (syn-Form).

2,52 g 7-Amino-3-(1-pyridinium)-methyl-3-cephem-4-carbonat, 30 ml Tetrahydrofuran, 0,64 ml Trifluoressigsäure, 9,25 ml Bis-trimethylsilylacetamid und 30 ml Acetonitril werden in dieser Reihenfolge bei 20 °C zusammengegeben und 5 bis 10 Min. gerührt. Die dann klare Lösung wird auf − 50 °C abgekühlt und 3,24 g (= 2,81 g Reinsubstanz) des im Beispiel 13b beschriebenen Säurechlorid-hydrochlorids zugegeben, Anschließend wird das Kältebad entfernt und 2 Std. bei Raumtemperatur gerührt. Der vorhandene Niederschlag wird abgesaugt, mit Tetrahydrofuran/Acetonitril (1/1) und mit Ether gewaschen und getrocknet.

Ausbeute : 2,7 g (A).

Die Rohmutterlauge wird in 600 ml Ether gegossen, der ausgefallene Niederschlag abgesaugt, in 25 ml Eiswasser gelöst und mittels 300 ml EtOH eine zweite Fraktion ausgefällt.

Ausbeute : 1,5 g (B).

NMR (Subst. A) (250 MHz ; δ ; in $CD_3OD/D_2O$ bez. a. MeOH) ; 9,13 (d ; 2H) u. 8,63 (t ; 1H) u. 8,15 (m ; 2H) Pyridinium ; 7,06 (s ; 1H ; Thiazolin-5-) ; 5,92 (d ; 1H ; J = 5 Hz ; β-Lactam) ; 5,73 u. 5,35 (AB ; 2H ; $Cu_2$-Pyridinium) ; 5,26 (d ; 1H ; J = 5 Hz ; β-Lactam) ; 2,07 (m ; 2H ; Perhydrodiazin-5-C).

## Ansprüche

1. Cephalosporine der allgemeinen Formel III, in syn-Konfiguration und in kristalliner, reiner Form,

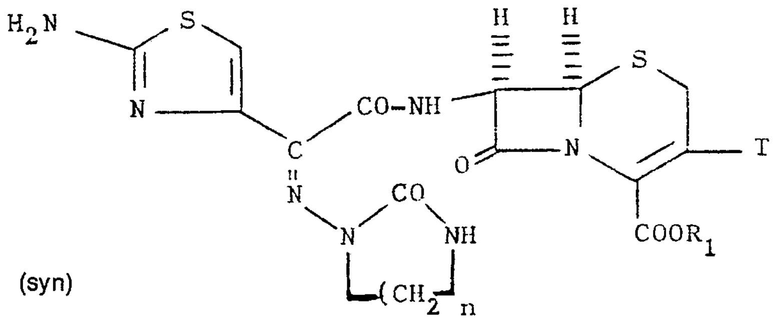

(syn)

(III)

in der
n 2 oder 3
$R^1$ Natrium oder, wenn ein anderer Molekülteil eine positive Ladung enthält, eine negative Ladung darstellt und
T $-CH_2-O-CO-CH_3$, $-CH_2O-CO-NH_2$ oder

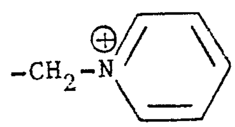

bedeutet.

2. Verbindung nach Anspruch 1, dadurch gekennzeichnet, daß n = 2, $R_1$ Natrium und T den Rest $-CH_2-O-CO-CH_3$ darstellt.

3. Verfahren zur Herstellung von Cephalosporinen der allgemeinen Formel III in syn-Konfiguration und in kristalliner, reiner Form

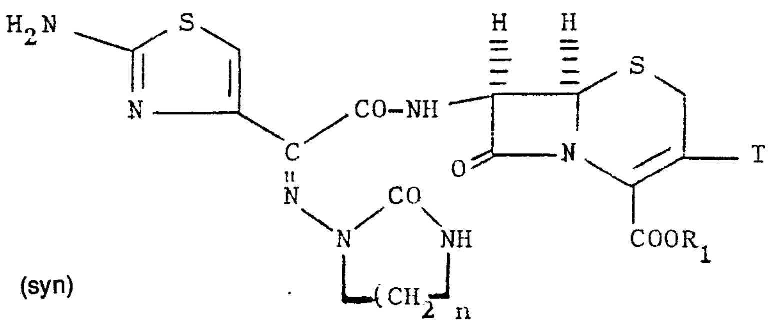

(syn)

(III)

wobei
n 2 oder 3
$R^1$ Natrium oder, wenn ein anderer Molekülteil eine positive Ladung enthält, eine negative Ladung darstellt und
T $-CH_2-O-CO-CH_3$, $-CH_2-O-CO-NH_2$ oder

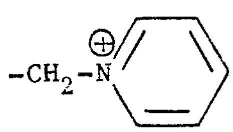

bedeutet, dadurch gekennzeichnet, daß man ein entsprechendes 7-Aminocephalosporansäurederivat, in dem $R_1$ und T die obengenannte Bedeutung haben, gegebenenfalls nach Silylierung der Carboxylgruppe und der 7-Amino-gruppe, mit einem Säurechlorid der allgemeinen Formel IV

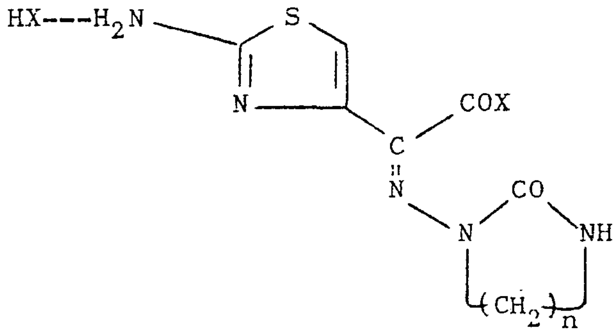

(IV)

in der

n die oben angegebene Bedeutung hat und

X Chlor bedeutet,

in Abwesenheit einer Base umsetzt und gegebenenfalls das erhaltene Produkt umkristallisiert.

4. Verbindungen der allgemeinen Formel IV

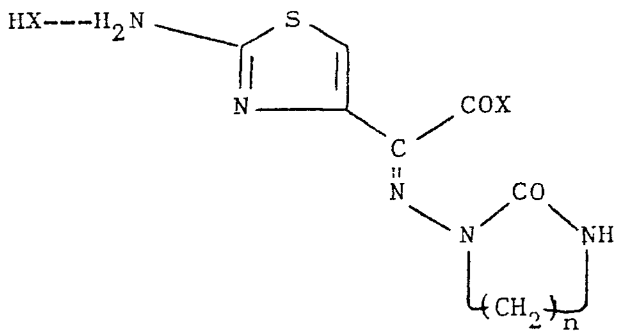

(IV)

in der X Chlor bedeutet und n für 2 oder 3 steht.

5. Verfahren zur Herstellung der Verbindungen gemäß Anspruch 4, dadurch gekennzeichnet, daß man Aminothiazolylglyoxylsäure

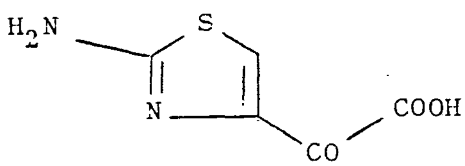

mit der Hydrazinverbindung

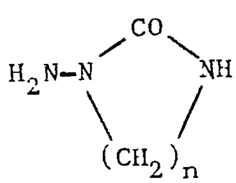

worin n die in Anspruch 3 angegebene Bedeutung hat zur Säure V umsetzt

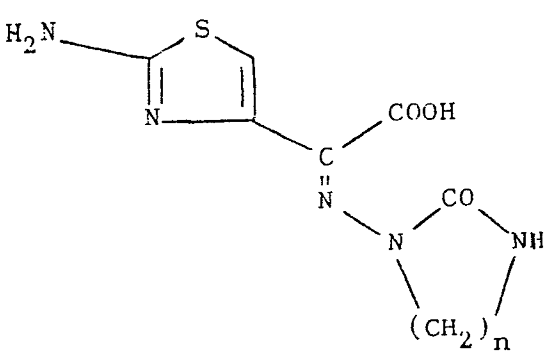

(V)

worin n die oben angegebene Bedeutung hat, und diese mit einem Chlorierungsmittel umsetzt, wobei die Umsetzung der Säure V mit Thionylchlorid als Chlorierungsmittel in Acetonitril oder einem niederen Nitroaliphaten durchgeführt wird und wobei Thionylchlorid im molaren Überschuß von mindestens 3 : 1 über die Säure V eingesetzt wird und wobei ferner das Reaktionsgemisch vor der Zugabe des Thionylchlorids auf einen Wassersgehalt gebracht wird, der in einem Molverhältnis von Säure V/$H_2O$ von 1 : 2 bis 1 : 4 im Falle von n = 2 und einem Molverhältnis von 1 : 0,4 bis 1 : 1,7 im Falle von n = 3 entspricht.

**Claims**

1. Cephalosporins of the general formula III, in syn-configuration and in the crystalline, pure form

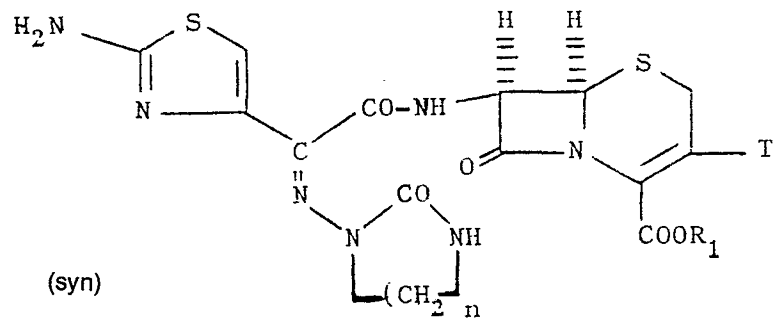

(syn)

(III)

in which

n represents 2 or 3,

$R^1$ represents sodium or, if another part of the molecule contains a positive charge, a negative charge and

T denotes $-CH_2-O-CO-CH_3$, $-CH_2-O-CO-NH_2$ or

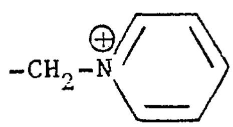

2. Compound according to Claim 1, characterised in that n = 2, $R_1$ represents sodium and T represents the radical $-CH_2-O-CO-CH_3$.

3. Process for the preparation of cephalosporins of the general formula III, in syn-configuration and in the crystalline, pure form

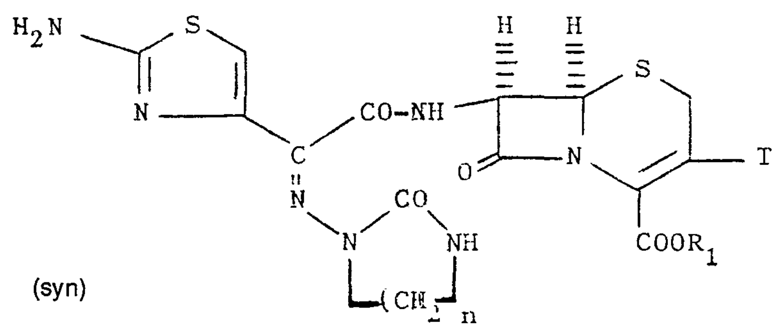

(syn)

(III)

in which

n represents 2 or 3,

$R^1$ represents sodium or, if another part of the molecule contains a positive charge, a negative charge and

T denotes $-CH_2-O-CO-CH_3$, $-CH_2-O-CO-NH_2$ or

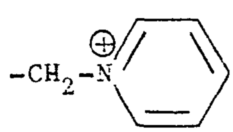

characterised in that a corresponding 7-aminocephalosporanic acid derivative in which $R_1$ and T have the

abovementioned meaning is reacted, if appropriate after silylation of the carboxyl group and the 7-amino group, with an acid chloride of the general formula IV

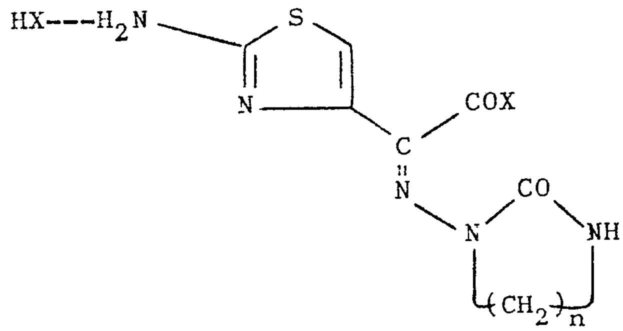

(IV)

in which
n has the meaning given above and
X denotes chlorine,
in the absence of a base and, if appropriate, the product obtained is recrystallised.

4. Compounds of the general formula IV

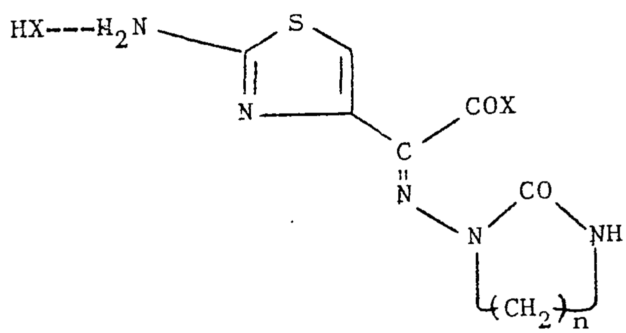

(IV)

in which
X denotes chlorine and
n represents 2 or 3.

5. Process for the preparation of the compounds according to Claim 4, characterised in that aminothiazolylglyoxylic acid

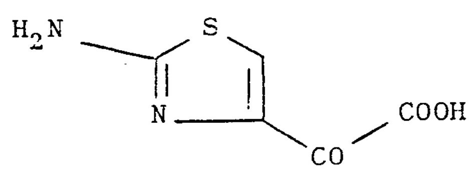

is reacted with the hydrazine compound

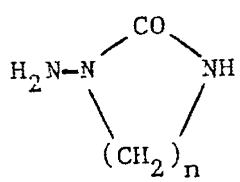

wherein n has the meaning given in Claim 3, to give the acid V

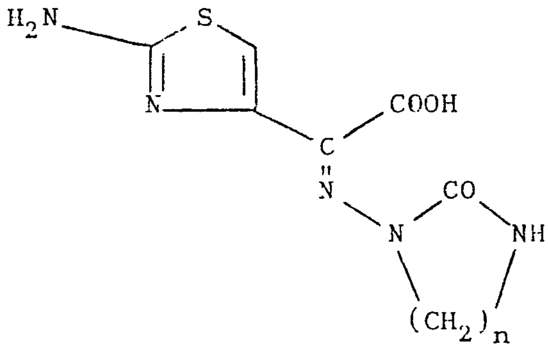

(V)

wherein n has the abovementioned meaning, and this acid is reacted with a chlorinating agent, the reaction of the acid V with thionyl chloride as the chlorinating agent being carried out in acetonitrile or a lower nitroaliphatic, thionyl chloride being employed in a molar excess of at least 3 : 1 relative to the acid V and, furthermore, before adding the thionyl chloride, the reaction mixture being brought to a water content which corresponds in a molar ratio of acid V/$H_2O$ of 1 : 2 to 1 : 4 where n = 2, and a molar ratio of 1 : 0.4 to 1 : 1.7 where n = 3.

**Revendications**

1. Céphalosporines de formule générale III dans la configuration syn et sous forme cristalline pure

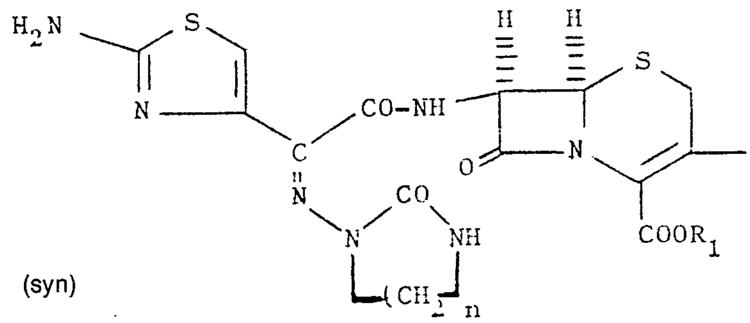

(syn) (III)

dans laquelle

n représente 2 ou 3,

$R_1$ représente le sodium ou, lorsqu'une autre fraction de la molécule contient une charge positive, il représente une charge négative, et

T représente $—CH_2—O—CO—CH_3$, $—CH_2—O—CO—NH_2$ ou

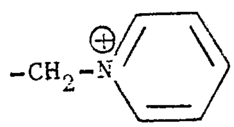

2. Composé suivant la revendication 1, caractérisé en ce que n = 2, $R_1$ représente le sodium et T représente le radical $—CH_2—O—CO—CH_3$.

3. Procédé de préparation de céphalosporines de formule générale III dans la configuration syn et sous forme cristalline pure

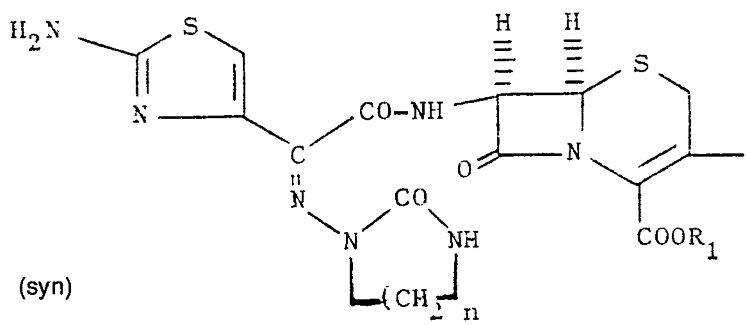

(syn) (III)

où

n représente 2 ou 3,

$R_1$ représente le sodium ou, lorsqu'une autre fraction de la molécule contient une charge positive, il représente une charge négative, et

T représente $—CH_2—O—CO—CH_3$, $—CH_2—O—CO—NH_2$ ou

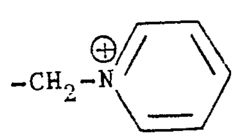

caractérisé en ce qu'on fait réagir un dérivé correspondant d'acide 7-aminocéphalosporanique dans

lequel $R_1$ et T ont les significations indiquées ci-dessus, éventuellement après silylation du groupe carboxy et du groupe 7-amino, avec un chlorure d'acide de formule générale IV

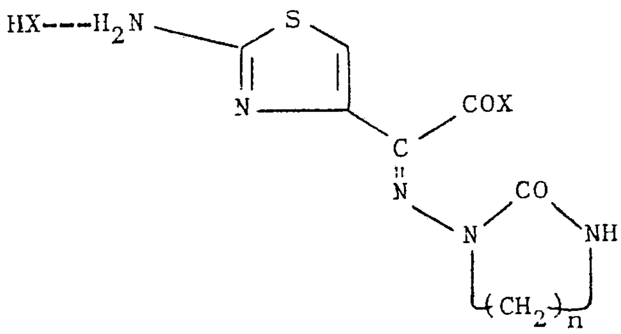

(IV)

dans laquelle

n a la signification indiquée ci-dessus, et

X représente le chlore,

en absence d'une base et on recristallise éventuellement le produit obtenu.

4. Composés de formule générale IV

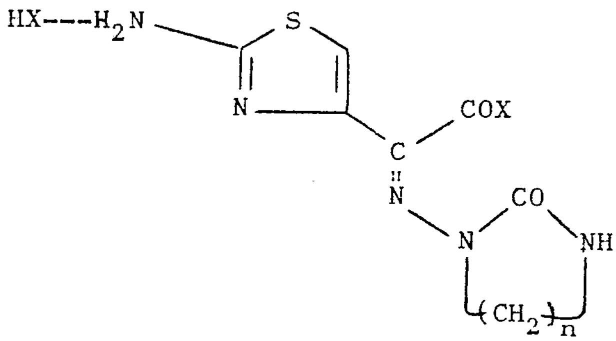

(IV)

dans laquelle X représente le chlore et n représente 2 ou 3.

5. Procédé de préparation des composés suivant la revendication 4, caractérisé en ce qu'on fait réagir l'acide aminothiazolyl-glyoxylique

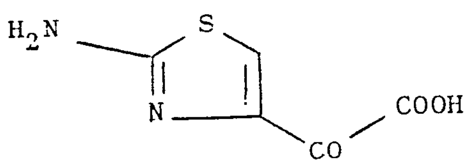

avec le composé d'hydrazine

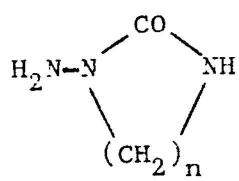

où n a la signification indiquée dans la revendication 3, pour obtenir l'acide V

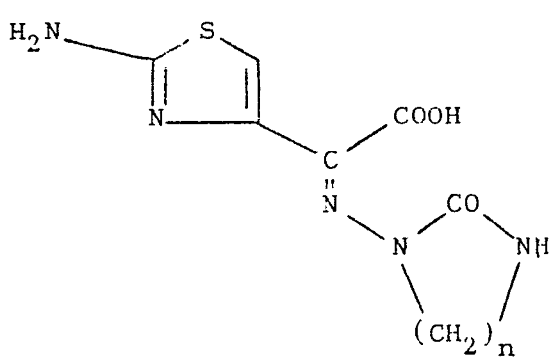

(V)

où n a la signification indiquée ci-dessus, et l'on fait réagir cet acide avec un agent de chloration, la réaction de l'acide V avec le chlorure de thionyle comme agent de chloration étant effectuée dans de l'acétonitrile ou dans un hydrocarbure nitroaliphatique inférieur, le chlorure de thionyle étant utilisé en un excès molaire d'au moins 3 : 1 vis-à-vis de l'acide V tandis que, en outre, avant l'addition du chlorure de thionyle, le mélange réactionnel est porté à une teneur en eau qui correspond à un rapport molaire acide V/$H_2O$ de 1 : 2 à 1 : 4 lorsque n = 2 et à un rapport molaire de 1 : 0,4 à 1 : 1,7 lorsque n = 3.

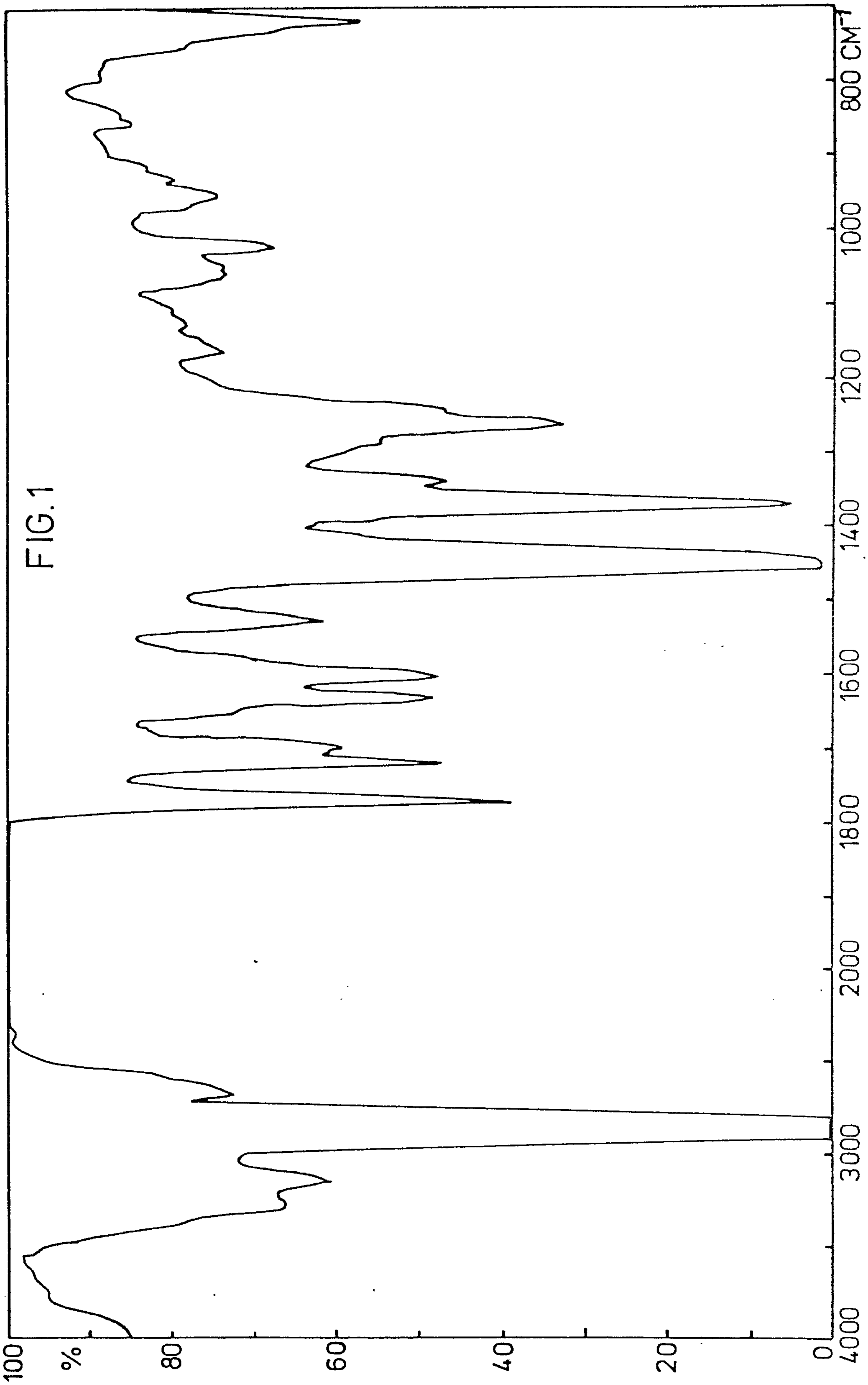
100
%
80
60
40
20
0
4000
3000
2000
1800
1600
1400
1200
1000
800 CM-1

FIG.1

1

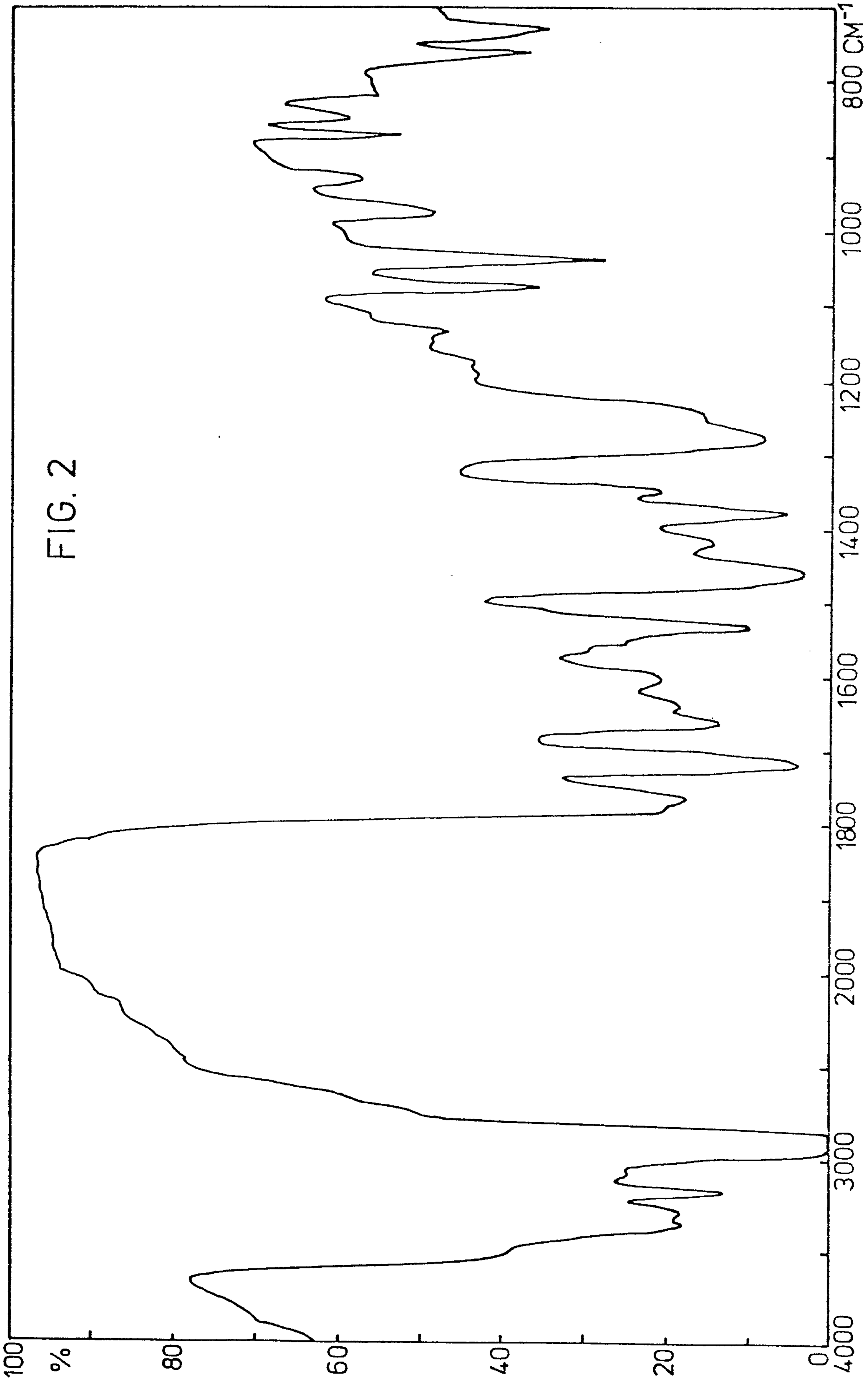
100
%
80
60
40
20
0
4000
3000
2000
1800
1600
1400
1200
1000
800 CM-1

FIG. 2